(19) Europäisches Patentamt
European Patent Office
Office européen des brevets

(11) EP 0 887 331 B1

(12) EUROPÄISCHE PATENTSCHRIFT

(45) Veröffentlichungstag und Bekanntmachung des Hinweises auf die Patenterteilung:
**31.10.2001 Patentblatt 2001/44**

(51) Int Cl.[7]: **C07C 41/08**, B01J 23/06, C07C 41/54

(21) Anmeldenummer: **98110002.7**

(22) Anmeldetag: **02.06.1998**

# (54) Verfahren zur Addition von Hydroxylgruppen enthaltenden Verbindungen an Alkine oder Allene

Process for the addition of hydroxyl group-containing compounds to alkynes and allenes

Procédé pour l'addition de composés contenant un groupe hydroxyle sur les alcynes ou les allènes

(84) Benannte Vertragsstaaten:
**CH DE FR GB IT LI**

(30) Priorität: **23.06.1997 DE 19726666**

(43) Veröffentlichungstag der Anmeldung:
**30.12.1998 Patentblatt 1998/53**

(73) Patentinhaber: **BASF AKTIENGESELLSCHAFT
67056 Ludwigshafen (DE)**

(72) Erfinder:
- **Teles, Joaquim Henrique Dr.
67122 Altrip (DE)**
- **Rieber, Norbert, Dr.
68259 Mannheim (DE)**
- **Breuer, Klaus, Dr.
67122 Altrip (DE)**
- **Rieker, Christopher William, Dr.
67454 Hassloch (DE)**
- **Demuth, Dirk, Dr.
68161 Mannheim (DE)**
- **Hibst, Hartmut, Prof. Dr.
69198 Schriesheim (DE)**
- **Hagemeyer, Alfred, Dr.
48431 Rheine (DE)**

(74) Vertreter: **Isenbruck, Günter, Dr. et al
Patent- und Rechtsanwälte,
Bardehle-Pagenberg-Dost-Altenburg-Geissler-Isenbruck
Theodor-Heuss-Anlage 12
68165 Mannheim (DE)**

(56) Entgegenhaltungen:
**CH-A- 239 752**

- **DATABASE WPI Week 7610 Derwent Publications Ltd., London, GB; AN 76-18332x XP002080699 & SU 449 034 A (M.A. GLIKIN) , 18. Juli 1975**
- **CHEMICAL ABSTRACTS, vol. 112, no. 7, 12. Februar 1990 Columbus, Ohio, US; abstract no. 54986a, O. N. TEMKIN: "A catalyst for the manufacture of methyl and ethyl isopropenyl ethers" Seite 687; Spalte 1; XP002080414 & DD 267 629 A**
- **CHEMICAL ABSTRACTS, vol. 112, no. 7, 12. Februar 1990 Columbus, Ohio, US; abstract no. 54987b, O. N. TEMKIN: "A catalyst for the manufacture of methyl and ethyl isopropenyl ether" Seite 687; Spalte 1; XP002080415 & DD 265 289 A**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 887 331 B1

## Beschreibung

Die Erfindung betrifft ein Verfahren zur Addition von Hydroxylgruppen enthaltenden Verbindungen an Alkine oder Allene unter Bildung von Aldehyden und Ketonen oder deren Derivaten in Form von Enolethern oder Acetalen bzw. Ketalen in Gegenwart eines amorphen Zink- oder Cadmiumsilikat-Katalysators. Ferner betrifft die Erfindung ein neues Verfahren zur Herstellung eines Zink- oder Cadmiumsilikats und den so erhaltenen Katalysator.

Die Addition von Hydroxylgruppen enthaltenden Verbindungen an Alkine bzw. Allene wird fast ausnahmsweise mit homogen gelösten Katalysatoren durchgeführt, z.B. mit Säuren, Basen und Übergangsmetallkomplexen (vgl. Houben-Weyl, Methoden der organischen Chemie, Bd. 6/3, S. 233, S. 90, Bd. 5/2a, S. 738, Bd. 6/1d, S. 136 und Bd. 7/a, S. 816).

Die Säurekatalyse ist meistens auf die Addition an aktivierte, elektronenreiche Alkine (wie Acetylenether, R-C≡C-OR', Acetylenthioether, R-C≡C-SR' und Acetylenamine, $R\text{-}C\equiv C\text{-}NR'_2$) beschränkt.

Basenkatalysiert (in Gegenwart von KOH oder Alkoholat) können Alkohole in der Flüssigphase auch an nicht aktivierte Alkine addiert werden. Dies ist die gebräuchlichste Methode; allerdings bedarf die Reaktion hoher Temperaturen und Drücke und die Raumzeitausbeute ist relativ gering. Typischerweise benötigt man für die basenkatalysierte Vinylierung eines Alkoholes zwischen 6 und 10 h Verweilzeit bei ca. 160°C und 18 bis 20 bar Druck.

Die Addition kann auch durch Übergangsmetallkomplexe in der Flüssigphase katalysiert werden. Für die Addition von Alkoholen eignen sich insbesondere Quecksilber(II)- oder Gold(I)-Salze, während für die Addition von Carbonsäuren und Phenolen Zink- und Cadmiumsalze bevorzugt werden.

Die Addition von Carbonsäuren (insbesondere Essigsäure und Propionsäure) an Acetylen kann auch in der Gasphase in Gegenwart entsprechender Zinkcarboxylate (auch basischer Zinkcarboxylate gemäß CH 239 752) auf oberflächenreichen Trägern als Katalysatoren durchgeführt werden.

Schließlich ist auch bereits die Addition von Methanol an Propin bzw. Propadien in der Gasphase in Gegenwart von Zinkoxid auf Aktivkohle oder Silikagel in DD 265 289 bzw. von Zinknitrat auf Aktivkohle oder Silikagel in DD 267 629 beschrieben.

Alle diese Verfahren des Standes der Technik haben Nachteile. Sie haben entweder nur ein beschränktes Anwendungsgebiet oder benötigen, wie die basenkatalysierte Addition, hohe Drücke und Temperaturen, was zu Sicherheitsproblemen führen kann, oder sie haben nur eine geringe Raumzeitausbeute. Homogen gelöste Übergangsmetallkatalysatoren sind oft nach einer geringen Anzahl von Zyklen desaktiviert und sind außerdem schwierig zurückzuführen. Heterogene Katalysatoren für die Addition an Alkine bzw. Allene sind bisher nur selten beschrieben worden. Zink- bzw. Cadmiumcarboxylate auf Aktivkohle katalysieren lediglich die Addition von Carbonsäuren (z.B. Essigsäure oder Propionsäure) an Acetylen. Der o.g. Katalysator auf der Basis von Zinkoxid auf Aktivkohle oder Silicagel (DD 265 289) ist zwar in der Lage die Addition von Alkoholen (Methanol oder Ethanol) an Propin bzw. Propadien mit guter Selektivität (90 bis 96 %) zu katalysieren, die katalytische Aktivität ist aber relativ gering und die benötigten Reaktionstemperaturen und Kontaktzeiten sind hoch. Zinknitrat auf Aktivkohle oder Silicagel ist bei gleicher Temperatur ungefähr eine Größenordnung weniger aktiv als das Zinkoxid und die Selektivität ist viel geringer (max. 70 %).

Es bestand daher die Aufgabe, einen möglichst bei niedrigen Temperaturen aktiven und selektiven heterogenen Katalysator für die Addition von Hydroxylgruppen enthaltende Verbindungen an Alkine, Allene oder Gemische davon vorzuschlagen.

Diese Aufgabe wurde erfindungsgemäß gelöst mit einem Verfahren zur Herstellung von Verbindungen der Formeln I bzw. II

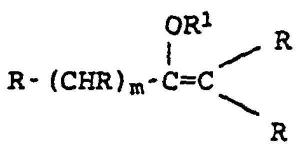 I 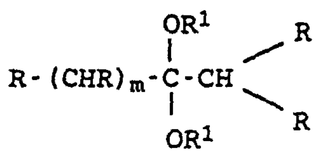 II

in denen $R^1$ Wasserstoff oder einen aliphatischen, cycloaliphatischen, araliphatischen, aromatischen oder heterocyclischen Rest oder einen Acylrest bedeutet, wobei diese Reste weitere Substituenten, die nicht mit Acetylenen oder Allenen reagieren, tragen können, die Reste R unabhängig voneinander für Wasserstoff, oder aliphatische, cycloaliphatische, araliphatische, aromatische oder heterocyclische Reste stehen, die unter Bildung eines Ringes miteinander verbunden sein können, und m für 0 oder 1 steht, durch Addition von Verbindungen der Formel III

$$R^1OH \qquad III$$

an Acetylene oder Allene der Formeln IV bzw. V

$$R\text{-}C\equiv C\text{-}R \qquad IV$$

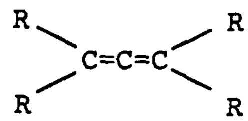

V

wobei $R^1$ und R die oben angegebenen Bedeutungen haben, in der Gasphase bei erhöhter Temperatur in Gegenwart eines heterogenen, ein Silikat enthaltenden Katalysators, bei dem man einen Katalysator verwendet, der als aktiven Bestandteil ein röntgenamorphes Zink- oder Cadmium-Silikat mit einem Gehalt an Zink oder Cadmium, berechnet als Oxid, von 1 bis 40 Gew.-% und z.B. eine BET-Oberfläche von 10 bis 1000 $m^2/g$ aufweist, erhältlich durch Aufbringen eines Zink- oder Cadmiumsalzes einer organischen aciden Verbindung, das bei Temperaturen unterhalb von 400°C zersetzbar ist, vorzugsweise der Formiate, Acetylacetonate und Acetate, auf amorphe Kieselsäure und Formierung des Katalysators bei Temperaturen von 50 bis 500°C, in Gegenwart einer Hydroxylgruppen enthaltenden Verbindung ausgewählt aus der Gruppe bestehend aus Wasser, Alkanolen mit 1 bis 10 C-Atomen, Diolen oder Polyolen mit 2 bis 6 C-Atomen und 2 bis 3 OH-Gruppen oder Carbonsäuren mit 1 bis 6 C-Atomen.

Für die Formierung des Katalysators verwendet man zweckmäsig das gleiche Alkanol $R^1OH$, das man an das Acetylen bzw. Allen addiert. Die erfindungsgemäß zu verwendenden Katalysatoren können mit bis zu 80, vorzugsweise mit bis zu 50 und insbesondere mit bis zu 20 Molprozent noch mit weiteren Metallen dotiert sein, ausgewählt aus der Gruppe (A) bestehend aus Natrium, Kalium, Lithium, Cäsium, Beryllium, Magnesium, Calzium, Strontium, Barium, Mangan, Eisen, Kobalt, Nickel und Kupfer, und der Gruppe (B), bestehend aus Titan, Zirkon, Hafnium, Germanium, Zinn und Blei.

Nach einer bevorzugten Ausführungsform des Verfahrens verwendet man amorpher Zinksilikat und stellt 2-Methoxypropen durch Addition von Methanol an Propin und/oder Allen her.

Als Edukte für die erfindungsgemäße Umsetzung kommen beliebige Alkine oder Allene oder Gemische davon in Betracht. In der Regel wird man jedoch technisch leicht zugängliche Acetylene und Allene mit 2 bis 8 C-Atomen, bzw. 3 bis 8 C-Atomen verwenden. Die Hydroxylgruppen enthaltende Verbindung $R^1OH$ kann Wasser, ein beliebiger Alkohol, ein Phenol oder eine Carbonsäure sein. Im allgemeinen kommen vor allem Alkohole, besonders Alkanole mit 1 bis 16 C-Atomen, einkernige Phenole und niedermolekulare Carbonsäuren, z.B. mit 1 bis 16 C-Atomen, in Betracht.

Die Addition der Hydroxylgruppen enthaltenden Verbindungen erfolgt in Gegenwart des heterogen vorliegenden Katalysators in der Gasphase entweder über einem Festbett oder in einem Wirbelbett bei Temperaturen von 50 bis 400°C, vorzugsweise 100 bis 250°C und besonders bevorzugt 120 bis 200°C und Drücken von 0,1 bis 100 bar, insbesondere 0,5 bis 20 bar (alle Drücke bezogen auf Summe der Partialdrücke der Edukte).

Gegebenenfalls kann das Reaktionsgemisch aus Gründen der Betriebssicherheit und der besseren Wärmeabfuhr mit Inertgasen wie Stickstoff, Argon, niedermolekularen Alkanen oder Olefinen verdünnt werden.

Das molare Verhältnis zwischen der Hydroxylgruppen enthaltenden Komponente und Alkin bzw. Allen kann zwischen 0,01 und 100 liegen. Bevorzugt wird der Bereich zwischen 0,1 und 5, und besonders bevorzugt wird der Bereich zwischen 0,7 und 1,3 gewählt.

Durch die Reaktionsbedingungen kann die Selektivität der Reaktion bezüglich der Mono- bzw. Diadditionsprodukte gesteuert werden. Niedrige Verhältnisse zwischen der Hydroxylgruppen enthaltenden Komponente und Alkin bzw. Allen sowie hohe Temperaturen und niedrige Partialdrücke der Reaktanden führen zur bevorzugten Bildung der Monoadditionsprodukte während hohe Verhältnisse zwischen der Hydroxylgruppen enthaltenden Komponente und Alkin bzw. Allen sowie niedrige Temperaturen und hohe Partialdrücke der Reaktanden die Bildung der Bisadditionsprodukte begünstigen. Beispielsweise kann aus Propin oder Allen mit Methanol je nach Reaktionsbedingungen selektiv 2-Methoxypropen oder 2,2-Dimethoxypropan gebildet werden

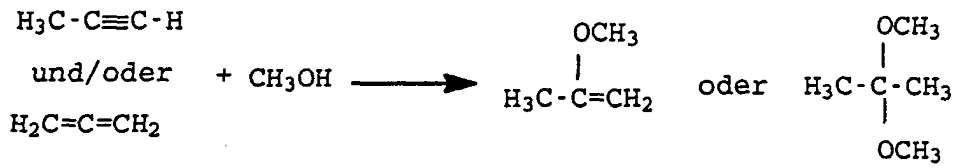


Zur Herstellung des Katalysators wird der amorphe $SiO_2$-Träger zweckmäßig mit einer Lösung der Zink- oder Cadmiumsalze einer organischen aciden Verbindung, die bei Temperaturen unterhalb von 400°C zersetzbar sind, imprägniert. Eine Vermischung des trockenen oder in den Alkoholen $R^1OH$ suspendierten Salze mit dem Träger ist zwar möglich aber im allgemeinen weniger vorteilhaft.

Der $SiO_2$-Träger ist zumindest weitgehend amorph, hat in der Regel eine BET-Oberfläche zwischen 10 und 1000 $m^2/g$, bevorzugt 100 bis 500 $m^2/g$, eine Wasseraufnahmekapazität von 0.1 bis 2 ml/g, bevorzugt von 0.7 bis 1.3 ml/g und kann als Pulver oder als fertiger Formkörper eingesetzt werden. Der Träger kann auch vor der Imprägnierung kalziniert werden. Bevorzugt wird der Träger aber nicht kalziniert.

Von den oben genannten Salzen sind die Zinksalze vor den Cadmiumsalzen bevorzugt. Von den Zinksalzen kommen besonders die in niederen Alkoholen und/oder Wasser löslichen Zink (II)-Salze, wie Zinkformiat, Zinkacetylacetonat und insbesondere das Zinkacetat in Betracht.

Besonders bevorzugt wird für die Imprägnierung eine ammoniakalische Zink (II)acetat-Lösung verwendet. Die Tränkung erfolgt nach an sich bekannten Methoden der Katalysatorherstellung. Wenn aus Löslichkeitsgründen erforderlich, kann die Beladung mit Zink auch in mehreren aufeinander folgenden Tränkschritten erfolgen.

Wenn der Träger als Pulver eingesetzt wird, kann er vor der Formierung durch Formgebung (z.B. durch Mischen, Kneten und Verstrangen oder Tablettieren) in die gewünschte Form gebracht werden.

Zur Erhöhung des Porenvolumens können bei der Formgebung auch Porenbildner zugesetzt werden (z.B. Superabsorber wie Lutexal P® (Firma BASF AG) oder Walocel® (Methylcellulose/Kunstharz-Kombination, Firma Wolff, Walsrode AG)).

Alternativ kann auch ein anderer Träger z.B. $Al_2O_3$ zusammen mit einer Siliziumoxid-Vorläuferverbindung (z. B. $Si(OR)_4$) und mit einem Zinksalz imprägniert werden.

Die Zink- bzw. Cadmiumbeladung kann in weiten Grenzen z.B. zwischen 1 und 40 Gew.-%, berechnet als Oxid, variieren. Bevorzugt werden Zink- bzw Cadmiumgehalte zwischen 7 und 30 Gew.-% und besonders bevorzugt zwischen 10 und 25 Gew.-%. Der so erhaltene, noch nicht aktive Präkatalysator kann dann bei einer Temperatur von maximal 600°C an Luft oder unter Inertgas kalziniert werden. Bevorzugt werden Kalziniertemperaturen zwischen 80 und 300°C. Besonders bevorzugt ist die Kalzinierung zwischen 120 und 250°C an Luft. Dabei wird zweckmäßig bei der Calcinierung darauf geachtet, daß man Temperatur und Verweilzeit so wählt, daß das Molverhältnis des noch vorhandenen Anions zu Zink bzw. Cadmium 0,1 nicht unterschreitet.

Nach der Herstellung des Präkatalysators, wird vor der Einbringung in den Reaktor oder in situ im Reaktor eine Formierung durchgeführt, bei der sich bevorzugt auf der Oberfläche des Katalysators die eigentliche Aktivphase ausbildet. Diese Gas/Festkörperreaktion wird bei einer Temperatur zwischen 80 und 400°C durch die Anwesenheit von Wasser, Alkoholen, bevorzugt niederen Alkoholen oder Carbonsäuren, bevorzugt niederen Carbonsäuren gefördert. Bevorzugt ist die Formierung des Katalysators zwischen 100 und 250°C in einem wasser- oder methanolhaltigen Gasgemisch und besonders bevorzugt zwischen 130 und 200°C mit einem methanolhaltigen Gasgemisch im situ im Reaktor, in dem die Umsetzung mit dem Alkin oder Allen stattfindet. Zweckmäßigerweise wird der Präkatalysator zur Ausbildung der Aktivphase unter Reaktionsbedingungen mit einem Gemisch aus Methanol mit Propin und Allen und gegebenenfalls auch noch anderen inerten Komponenten, wie z.B. Propen oder Propan in Kontakt gebracht. Die Bildung der Aktivschicht wird durch das Ansteigen des Propin- und Allen-Umsatzes (nach ca. 5 bis 30 min, je nach Temperatur), durch das Ansteigen der Selektivität (nach 10 bis 300 min, je nach Temperatur) und durch das Abklingen der Konzentration von Methylacetat im Abgas bei Verwendung eines Präkatalysators aus Zinkacetat angezeigt. Ein stationärer Zustand und eine hohe Selektivität wird je nach Temperatur nach ca. 2 bis 20 Stunden erreicht.

Für die Charakterisierung der Katalysatorproben (frische, als auch Ausbauproben) wurden Standardmethoden verwendet. Die gemessene BET-Oberfläche (in der Regel zwischen 10 und 800 $m^2/g$) sowie die Härte sind bei dem jeweiligen Beispiel angegeben. Bevorzugt werden Katalysatoren mit BET-Oberflächen zwischen 100 und 400 $m^2/g$ verwendet. Weiterhin wurden die Proben mittels Pulverröntgendiffraktometrie (XRD) und Transmissionselektronenmikroskopie (TEM) eingehend untersucht. Mit beiden strukturaufklärenden Methoden war keine Fernordnung im Sinne einer kristallinen Struktur festzustellen, sämtliche Proben waren amorph. Die Verteilung des Zinks über dem Träger wurde an Schnitten im Elektronenmikroskop sowie in der Mikrosonde untersucht. Sämtliche Proben zeigten, auch nach Ausbau, daß der Katalysator eine weitgehend homogene Elementverteilung besitzt und kein oder nur wenig kristallines ZnO enthält. In der IR-Untersuchung (KBr-Preßling) zeigte der aktive Katalysator keine Acetat-Banden (diese sind im

Präkatalysator bei 1570, 1410, 670 und 610 $cm^{-1}$ noch sichtbar). Im $^{13}C$-CP-MAS-NMR sind auch keine Signale für Acetat mehr vorhanden. Im $^{29}Si$-CP-MAS-NMR zeigt der Katalysator nur die breite Bande bei -109 ppm typisch für das amorphe $SiO_2$ und eine Schulter bei -99 ppm (ca. 15 % der Intensität des Hauptpeaks). Die Elementaranalyse eines Zn-Acetat/$SiO_2$-Präkatalysators zeigte, daß das molare Verhältnis Acetat/Zn von der Kalzinierungstemperatur abhängt. Bei Raumtemperatur getrocknete Katalysatoren haben ein Acetat/Zn-Verhältnis von 1,8 bis 2. Nach der Kalzinierung im bevorzugten Temperaturbereich von 200 bis 250°C liegt das Acetat/Zn-Verhältnis zwischen 0,5 und 1. Bei höheren Temperaturen sinkt das Acetat/Zn-Verhältnis noch weiter und ebenso die katalytische Aktivität der daraus gebildeten Katalysatoren.

Der Katalysator kann für die Umsetzung fest angeordnet sein oder z.B. auch in einem Wirbelbett verwendet werden und dafür eine entsprechende Gestalt haben wie Splitt, Tabletten, Monolithe, Kugeln oder Extrudate (Stränge mit entsprechenden Querschnitten wie Vollstrang, Wagenrad, Stern oder Ring).

(a) Allgemeine Umsetzungsbedingungen

Die katalytischen Umsetzungen gemäß Fig 1. wurden in einem gradientenfreien CSTR (Continuously Stirred Tank Reactor) (A) mit einem Volumen von 1740 ml und einem Katalysatorvolumen von ca. 90 ml, modifiziert für heterogene Gasphasenreaktionen durchgeführt. Der Reaktor hatte einen Innendurchmesser von ca. 108 mm und eine Höhe von ca. 200 mm und wurde mittels einer an der Innenwand angebrachten elektrischen Heizspirale beheizt. In der Mitte des Reaktors war ein kleiner Zylinder aus Metall angebracht (∅ ca. 64 mm, Höhe ca 150 mm), der auf halber Höhe (ca. 85 mm unterhalb der Oberkante) mit einem Drahtgitter versehen war. Auf dieses Drahtgitter wurde der Katalysator locker aufgeschüttet. Auf dem Deckel des Reaktors war eine flache (∅ ca. 100 mm, Höhe ca. 20 mm) Turbine angebracht, die mit 1500-2000 Upm angetrieben wurde. An der Reaktorachse waren auf verschiedenen Höhen insgesamt 6 Thermoelemente zur Temperaturkontrolle angebracht.

Die Edukte wurden unter Druck mittels HPLC-Pumpen dosiert, kurz vor dem Reaktor gemischt und in den Reaktorraum entspannt.

Das Alkin bzw. Allen (1 in Fig 1) wurden entweder in reiner Form zudosiert oder als Gemisch mit anderen inerten Komponenten verdünnt. In dem Fall von Propin und Allen wurde ein Gemisch mit anderen Kohlenwasserstoffen eingesetzt (Zusammensetzung: 30-43 Vol% Propin, 16-20 Vol% Allen, 20-45 Vol% Propen, 5-10 Vol% Isobutan und 2-6 Vol% Propan als Hauptkomponenten; alle anderen Komponenten unter 1 %. Dieses Gemisch wurde durch Destillation aus einem Seitenstrom eines Steamcrackers gewonnen). Der Alkoholkomponente (2 in Fig 1) wurden ca. 10 Gew.-% Cyclohexan als interner Standard für die GC-Analytik zudosiert.

Die Reaktion wurde isotherm bei Temperaturen von 120 bis 300°C und einer Zulaufrate von 0,5 bis 10 mmol/min Propin und/oder Allen und 0,5 bis 20 mmol/min MeOH durchgeführt. Der Reaktionsdruck betrug 1,1 bis 3,5 bar (absolut).

Die Gesamtgasmenge, bestehend aus Edukten, Inertgas und internem Standard, betrug in der Regel zwischen 4 und 60 Nl/h. Die GHSV (gas hourly space velocity), die definiert ist als

GHSV = Gasvolumen [Nl/h]/Katalysatorvolumen [l]

betrug zwischen 80 und 1200 $h^{-1}$. Die LHSV (liquid hourly space velocity), die definiert ist als

LHSV = Flüssigkeitsvolumen [Nl/h]/Katalysatorvolumen [l]

(hier gefördertes Volumen an Propin und MeOH Volumen) betrug zwischen 0,2 und 3 $h^{-1}$. Die Verweilzeit, definiert als Quotient aus dem Katalysatorvolumen [l] und der Gasmenge [Nl/s] betrug zwischen 3 und 40 s.

Die Reaktionsgase wurden nach Verlassen des Reaktors über eine beheizte Transferleitung (3) zu einem On-line Gaschromatographen (B) geführt und dort alle 2 h analysiert. Danach wurde der Gasstrom einer partiellen Kondensation (C) unterworfen und der bei Raumtemperatur nicht kondensierbare Anteil (6) wurde in regelmäßigen Abständen (ca. 12 h) mittels Off-line GC analysiert. Das Kondensat (5) wurde ebenfalls gesammelt und mittels Off-line GC analysiert.

Wenn nichts anderes vermerkt, wurden die Umsätze und Selektivitäten auf die Summe von Propin und Allen bezogen.

b) Methoden zur Charakterisierung der Katalysatoren

Für die Charakterisierung der Katalysatorproben (frische als auch Ausbauproben) wurden Standardmethoden

verwendet. Die gemessene BET-Oberfläche sowie die Härte sind bei dem jeweiligen Beispiel angegeben. Weiterhin wurden die Proben mittels Pulverröntgendiffraktometrie (XRD) und Transmissionselektronenmikroskopie (TEM) eingehend untersucht. Die Verteilung des Zinks über den Träger wurde mittels Schnittbildern im Elektronenmikroskop sowie in der Mikrosonde kontrolliert. Ausgewählte Proben wurden außerdem noch mittels IR, $^{13}C$- und $^{29}Si$-CP-MAS-NMR und EXAFS untersucht.

Die erfindungsgemäß erhältlichen Enolether der Formel I und die Dialkoxyverbindungen der Formel II sind wertvolle Zwischenprodukte zur Herstellung von Wirkstoffen und Riechstoffen. Insbesondere die Enolether sind begehrte Ausgangsstoffe z.B. zur Herstellung von γ,δ-ungesättigten Ketonen als Vorprodukte für die Herstellung von Isophytol.

Will man vor allem die Enolether gewinnen, kann man in an sich bekannter Weise die Verbindungen der Formel II durch Abspaltung von einem Mol $R^1OH$ in die entsprechenden Enolether der Formel I überführen. Dafür existieren zahlreiche aus DE-A-35 35 128, DE-A-37 22 891, DE-A- 38 04 162, Chemical Abstracts, Vol 94 (19); 156241 f und DE-A-19544450 bekannte Verfahren.

Beispiel 1

a) (Präkatalysator: Gehalt berechnet als Oxide 20 % ZnO, 80 % $SiO_2$)

Der Zn/$SiO_2$-Trägerkatalysator wurde durch Imprägnierung von röntgenamorphen $SiO_2$-Formkörpern (Kugeln mit Ø 3 - 6 mm) mit einer BET-Oberfläche von 358 $m^2/g$, einer Wasseraufnahmekapazität von 0.9 ml/g, und einer Stempelhärte von 43 N mit ammoniakalischer Zinkacetatlösung, hergestellt.

Dazu wurden 100 g $SiO_2$-Träger (Siligel, Fa. Solvay) mit 67.42 g $Zn(OAc)_2 \cdot 2H_2O$ (Merck) gelöst in 58 g 25 %iger $NH_4OH$-Lösung bei Raumtemperatur getränkt, der Präkatalysator anschließend 16 h bei 120°C getrocknet und dann bei 250°C 4 h unter Luft kalziniert.

Der Präkatalysator besaß eine BET-Oberfläche von 206 $m^2/g$ unc eine Härte von 64 N/Formkörper. Das Acetat/Zn-Verhältnis lag bei 0,56 mol/mol.

b) In die oben beschriebene Apparatur wurden ca. 90 ml des Präkatalysators eingefüllt. Propin/Allen-Gemisch (ca. 55 Vol%ig, 2.8 mmol/min) und Methanol (2.1 mmol/min; Gesamtzulauf mit inerten : 7.3 mmol/min; Verhältnis MeOH/Propin+Allen = 0.75) wurden dann mittels HPLC-Pumpen zudosiert. Die Reaktionstemperatur betrug 170°C und der Druck 1.2 bar (abs) bzw. der Partialdruck der Edukte 0,8 bar. Nach der vollständige Bildung des Katalysators (nach ca. 14 h Laufzeit) betrug der Umsatz 67 % (bezogen auf Propin und Allen) bzw. 90 % (Methanol). Als Hauptprodukte bildeten sich (Selektivitäten bzgl. Propin+Allen in Klammern) 2-Methoxypropen (87 %), 2,2-Dimethoxypropan (4 %), 1-Methoxypropen (4 %) und Aceton (2 %).

Für den fertig gebildeten Katalysator wurden nach dem Ausbau die folgenden Werte ermittelt: BET 206 $m^2/g$, Härte 64 N/Formkörper.

Beispiel 2

a) (Präkatalysator: Gehalt berechnet als Oxide 19.5 % ZnO, 0.5 % $Na_2O$, 80 % $SiO_2$)

Eine Tränklösung bestehend aus 131.46 g $Zn(OAc)_2 \cdot 2H_2O$ (Merck) und 2.08 g $Na(OAc) \cdot 3H_2O$ gelöst in einem Gemisch aus 160 g destillierten Wasser und 120 g 25 %iger $NH_4OH$-Lösung wurde in zwei Teile von je 195 ml geteilt, und 200 g $SiO_2$-Träger (Siliperl® AF125, Fa. Engelhard) wurden bei Raumtemperatur mit dem ersten Anteil getränkt. Der Präkatalysator wurde anschließend 16 h bei 120°C getrocknet, mit dem zweiten Anteil bei Raumtemperatur getränkt, anschließend 16 h bei 120°C getrocknet und dann bei 250°C 4 h unter Luft kalziniert. Der Präkatalysator besaß eine BET-Oberfläche von 212 $m^2/g$ und hatte eine Härte von 61 N/Formkörper. In die oben beschriebene Apparatur wurden ca. 90 ml des Präkatalysators eingefüllt und das Propin/Allen-Gemisch und Methanol dann mittels HPLC-Pumpen zudosiert. Die Reaktion wurde bei einer ersten Temperatureinstellung von 170°C so lange fortgeführt bis der aktive Katalysator vollständig gebildet war und Umsatz und Selektivität konstant waren. Danach wurde die Temperatur und die Zuläufe geändert. Die Ergebnisse sind in Tabelle 1 zusammengefaßt. Der Druck betrug bei allen Versuchen 1.2 bar (abs).

Abkürzungen: 2MP: 2-Methoxypropen; 22DMP: 2,2-Dimethoxypropan; 1MP: 1-Methoxypropen (cis und trans); 11DMP: 1,1-Dimethoxypropan.

Tabelle 1

| # | Temp. °C | Zuläufe/mmol/min | | | Umsatz | Selektivitäten/% | | | | |
|---|---|---|---|---|---|---|---|---|---|---|
| | | Propin/ Allen | Methanol | Gesamt | Propin/ Allen | 2MP | 22DMP | Aceton | 1MP | 11DMP |
| 2.1 | 170 | 2.91 | 3.64 | 10.13 | 76 % | 84 | 10 | 1 | 4 | 1 |
| 2.2 | 160 | 2.37 | 4.78 | 9.87 | 71 % | 65 | 30 | 1 | 4 | 0 |
| 2.3 | 150 | 2.33 | 4.77 | 9.79 | 67 % | 61 | 35 | 0 | 3 | 0 |
| 2.4 | 140 | 1.10 | 2.47 | 4.98 | 45 % | 51 | 44 | 1 | 3 | 0 |
| 2.5 | 130 | 0.75 | 2.48 | 4.21 | 39 % | 22 | 73 | 1 | 4 | 0 |

Für den nach dem Ende der Versuchsreihe ausgebauten Katalysator wurden die folgenden Werte ermittelt: BET 213 $m^2/g$, Härte 45 N/ Formkörper.

Beispiel 3

(Präkatalysator: Gehalt berechnet als Oxide: 15 % ZnO, 85 % $SiO_2$)

Die $SiO_2$Formkörper wurden wie in Beispiel 1 beschrieben in einer einstufigen ammoniakalischen Tränkung hergestellt: 100 g $SiO_2$-Träger (Siligel, Fa. Solvay) wurden mit 47.59 g $Zn(OAc)_2 \cdot 2\ H_2O$ (Merck) gelöst in 54 g 25 %iger $NH_4OH$-Lösung bei Raumtemperatur getränkt, der Präkatalysator anschließend 16 h bei 120°C getrocknet und dann bei 250°C für 4 h unter Luft kalziniert.
Der Präkatalysator besaß eine BET-Oberfläche von 246 $m^2/g$ und eine Härte von 47 N/Formkörper. In die oben beschriebene Apparatur wurden ca. 90 ml des Präkatalysators eingefüllt. Propin/Allen-Gemisch (ca. 55 Vol %ig, 2.6 mmol/min) und Methanol (2.1 mmol/min; Gesamtzulauf mit Inerten: 7.0 mmol/min; Verhältnis MeOH/(Propin+Allen) = 0.79) wurden dann mittels HPLC-Pumpen zudosiert. Die Reaktionstemperatur betrug 170°C und der Druck 1.2 bar (abs), bzw. der Parialdruck der Edukte 0,8 bar. Nach der vollständigen Bildung des Katalysators (ca. 14 h) betrug der Umsatz 69 % (für Propin/Allen) bzw. 86 % (für Methanol). Als Produkte bildeten sich (Selektivitäten in Klammern): 2-Methoxypropen (86 %); 2,2-Dimethoxypropan (4 %); cis und trans 1-Methoxypropen (4 %); Aceton (2 %); 1,1-Dimethoxypropan (1 %). Für den fertig gebildeten Katalysator wurden nach dem Ausbau die folgenden Werte ermittelt: BET 235 $m^2/g$, Härte 61 N/Formkörper.

Beispiel 4

(Präkatalysator: Gehalt berechnet als Oxide 25 % ZnO, 75 % $SiO_2$)

Die $SiO_2$ Formkörper wurden wie in Beispiel 1 beschrieben in einer einstufigen ammoniakalischen Tränkung präpariert. 100 g $SiO_2$-Träger (Siligel) wurden mit 89.89 g $Zn(OAc)_2 \cdot 2\ H_2O$ (Merck) gelöst in 55 g 25 %iger $NH_4OH$-Lösung getränkt, der Präkatalysator anschließend für 16 h bei 120°C getrocknet und dann bei 250°C für 4 h unter Luft kalziniert.
Der Präkatalysator besaß eine BET-Oberfläche von 189 $m^2/g$ und eine Härte von 56 N/Formkörper. In die oben beschriebene Apparatur wurden ca. 90 ml des Präkatalysators eingefüllt und das Propin/ Allen-Gemisch (ca. 55 Vol%ig, 2.6 mmol/min) und Methanol (2.1 mmol/min; Gesamtzulauf mit Inerten: 7.0 mmol/min; Verhältnis MeOH/(Propin+Allen) = 0.79) dann mittels HPLC-Pumpen zudosiert.

Die Reaktionstemperatur betrug 170°C und der Druck 1.2 bar (abs), bzw. der Partialdruck der Edukte 0,8 bar. Nach der vollständige Bildung des Katalysators (ca. 14 h) betrug der Umsatz 63 % (für Propin/Allen) bzw. 85 % (für Methanol). Als Produkte bildeten sich (Selektivitäten in Klammern): 2-Methoxypropen (87 %); 2,2-Dimethoxypropan (4 %); cis und trans 1-Methoxypropen (4 %); Aceton (2 %); 1,1-Dimethoxypropan (1 %).

Für den fertig gebildeten Katalysator wurden nach dem Ausbau die folgenden Werte ermittelt: BET 222 $m^2/g$, Härte von 73 N/Formkörper.

Beispiel 5

Verfährt man wie in Beispiel 1 oder Beispiel 2 beschrieben, ersetzt jedoch die dort verwendeten $SiO_2$-Träger durch Siliperl® AF 125 (Fa. Engelhard) oder die $SiO_2$-Träger D 11 - 10 oder D 11 - 13 (BASF Aktiengesellschaft) oder einen $SiO_2$-Träger, dessen Oberfläche hydrophobiert worden ist, erhält man vergleichbare Ergebnisse.

Beispiel 6

Zur Herstellung des Präkatalysators verfuhr man wie in Beispiel 1 beschrieben, führte die Tränkung jedoch ohne Zusatz von $NH_4OH$ aus.

Nach der vollständige Bildung des Katalysators (ca. 8 h) wurden folgende Selektivitäten beobachtet: 2-Methoxypropen: 82 %; Aceton: 6 %, 2,2-Dimethoxypropan: 4 %; cis und trans 1-Methoxypropen: 4 %, 1,1-Dimethoxypropan: 1 %. Der Propin/Allen-Umsatz betrug 77 %.
Für den fertig gebildeten Katalysator wurden nach dem Ausbau die folgenden Werte ermittelt: BET 215 $m^2/g$, Härte von 39 N/Formkörper.

Beispiel 7

(Präkatalysator: Gehalt berechnet als Oxide: 7,5 % CdO, 7,5 % ZnO, 85 % $SiO_2$)

Der Cd/Zn/$SiO_2$-Trägerkatalysator wurde durch Imprägnierung von röntgenamorphen $SiO_2$-Formkörpern (Kugeln mit ∅ 3 - 5 mm) mit einer BET-Oberfläche von 413 $m^2$/g, einer Wasseraufnahmekapazität von 0.99 ml/g und einer Härte von 29 N/Formkörper mit Cadmiumnitrat- und Zinkacetatlösung hergestellt. Dazu wurde eine Tränklösung bestehend aus 18.31 g $Cd(OAc)_2 \cdot 2\ H_2O$ (Merck) und 23.80 g $Zn(OAc)_2 \cdot 2\ H_2O$ (Merck) gelöst in 150 g destillierten Wasser in zwei Anteile von 95 ml und 85 ml geteilt und 100 g $SiO_2$-Träger (Siliperl® AF125, Fa. Engelhard) bei Raumtemperatur mit dem ersten Anteil getränkt, der Präkatalysator anschließend 16 h bei 120°C getrocknet, dann mit dem zweiten Anteil bei Raumtemperatur getränkt, anschließend. 16 h bei 120°C getrocknet und schließlich bei 500°C für 2 h unter Luft kalziniert.
Der Präkatalysator wies eine BET-Oberfläche von 253 $m^2$/g und Stempelhärte von 40 N/Formkörper auf.
In der oben beschriebene Apparatur wurden ca. 90 ml des Präkatalysators eingefüllt und das Propin/Allen-Gemisch (ca. 61 Vol%ig, 3.2 mmol/min) und Methanol (2.0 mmol/min; Gesamtzulauf mit Inerten: 7.5 mmol/min; Verhältnis MeOH/(Propin+Allen)= 0.65) dann mittels HPLC-Pumpen zudosiert. Die Reaktionstemperatur betrug 170°C und der Druck 1.2 bar (abs), bzw. der Partialdruck der Edukte 0,83 bar. Nach der vollständige Bildung des Katalysators (ca. 8 h) wurden folgenden Selektivitäten beobachtet: 2-Methoxypropen: 78 %; 2,2-Dimethoxypropan: 4 %; Aceton: 6 %; cis und trans 1-Methoxypropen: 3 %, 1,1-Dimethoxypropan: 1 %. Der Propin/ Allen-Umsatz betrug 61 %.
Für den fertig gebildeten Katalysator wurden nach dem Ausbau die folgenden Werte ermittelt: BET 241 $m^2$/g und eine Härte von 44 N/ Formkörper.

Beispiel 8

a) In situ Formierung des Katalysators bei 200°C (12,5 % ZnO)

500 g $SiO_2$-Pulver (D11-10, BASF Aktiengesellschaft), hergestellt durch Fällung von Kieselsäure mit einer BET-Oberfläche von 164 $m^2$/g wurden zuerst durch Kalzinierung bei 900°C (2 h)hydrophobiert. 100 g des hydrophobierten $SiO_2$-Pulvers wurden mit 3 g Kartoffelstärke, 2 g einer 25 %ige Ammoniaklösung und 140 g Wasser 2 h geknetet. Daraus wurden dann Stränge gepreßt (Preßdruck: 40 bar) die 16 h bei 120°C getrocknet und 6 h bei 550°C kalziniert wurden. Der Träger hatte einer BET-Oberfläche von 127 $m^2$/g, eine Wasseraufnahme von 1.0 ml/g und eine Härte <1 N/Strang. 75 g des $SiO_2$-Trägers wurden mit einer Lösung von 28.90 g $Zn(OAc)_2 \cdot 2\ H_2O$ (Merck) in 74 ml Wasser in einem Schritt imprägniert. Anschließend wurde der Präkatalysator 16 h bei 120°C getrocknet und 4 h bei 250°C an der Luft kalziniert.
Der Präkatalysator besaß eine BET-Oberfläche von 74 $m^2$/g und eine Härte von 7 N/Formkörper.
In die oben beschriebene Apparatur wurden ca. 90 ml des Präkatalysators eingefüllt, und das Propin/Allen-Gemisch (ca. 61 Vol%ig, 3.1 mmol/min) und Methanol (3.9 mmol/min; Gesamtzulauf mit Inerten: 9.2 mmol/min; Verhältnis MeOH/(Propin+Allen)= 1.24) wurde dann mittels HPLC-Pumpen zudosiert. Die Reaktionstemperatur betrug 200°C und der Druck 1.2 bar (abs), bzw. der Partialdruck der Edukte 0,91 bar

Nach einer Induktionszeit von ca. 40 min in dem keine Umsetzung stattfand setzte die katalytische Aktivität langsam ein. Direkt nach dem Einsetzen der katalytischen Aktivität bildeten sich fast ausschließlich Aceton und Methylacetat (das Acetat stammt aus der Zersetzung des Zinkacetats). Nach ca. 80 min hatte der Umsatz an Propin/Allen schon 35 % (ca. die Hälfte des Endwertes) erreicht und die Selektivität zu 2-Methoxypropen betrug 53 %. Nach ca. 150 min hatte der Umsatz an Propin/Allen schon 67 % (ca. 95 % des Endwertes) erreicht und die Selektivität zu 2-Methoxypropen betrug 85 %. Nach der vollständige Bildung des Katalysators (ca. 5 h) wurden folgenden Selektivitäten beobachtet: 2-Methoxypropen: 85 %; 2,2-Dimethoxypropan: 5 %; Aceton: 4 %; cis und trans 1-Methoxypropen: 4 %, 1,1-Dimethoxypropan: 1 %. Der Propin/ Allen-Umsatz betrug 71 %. Für den fertig gebildeten Katalysator wurden nach dem Ausbau die folgenden Werte ermittelt: BET 72 $m^2$/g; Härte 8 N/Formkörper.

b) Vorformierung des Katalysators bei 200°C

In die oben beschriebene Apparatur wurden ca. 90 ml desselben Präkatalysators eingefüllt. Während der ersten 4 Stunden wurde nur Methanol (3.9 mmol/min) zudosiert. Erst nach dieser Zeit wurde mit der Dosierung des Propin/Allen-Gemisch (ca. 61 Vol%ig, 3.1 mmol/min; Gesamtzulauf mit Inerten: 9.2 mmol/ min; Verhältnis MeOH/(Propin+Allen)= 1.31) begonnen. Die Temperatur im Reaktor wurde von Anfang an bei 200°C und der Druck auf 1.2 bar (abs), bzw. der Partialdruck der Edukte auf 0,91 bar gehalten.
Der Katalysator zeigte jetzt keine Induktionsperiode mehr. Ca. 10 Minuten nach dem Beginn der Propin/Allen-Dosierung betrug der Umsatz an Propin/Allen 53 %. Dieser Wert blieb dann weitgehend konstant (56 % Umsatz nach weiteren 3 Stunden). Die Selektivität zu 2-Methoxypropen blieb auch von Anfang an konstant. Drei Stunden nach dem Beginn der Propin/Allen-Dosierung wurden folgenden Selektivitäten beobachtet: 2-Methoxypropen: 80 %; 2,2-Dimethoxypropan: 8 %; Aceton: 5 %; cis und trans 1-Methoxypropen: 4 %, 1,1-Dimethoxypropan: 1 %. Für den fertig gebildeten Katalysator wurden nach dem Ausbau die folgenden Werte ermittelt: BET 87 $m^2/g$, Härte 1 N/Formkörper.

Beispiel 9

a) (Präkatalysator: Gehalt berechnet als Oxide: 20 % ZnO, 4 % $Cs_2O$ und 76 % $SiO_2$)

Der Zn/Cs/$SiO_2$-Trägerkatalysator wurde durch Imprägnierung von röntgenamorphen $SiO_2$-Formkörpern (Kugeln mit Ø 1,5 - 3,5 mm) mit einer BET-Oberfläche von 300 $m^2/g$, einer Wasseraufnahmekapazität von 0.9 ml/g, und einer Stempelhärte von 35 N/Formkörper mit Zinkacetat- und Cäsiumacetatlösung hergestellt. Dazu wurde eine Tränklösung bestehend aus 70.97 g $Zn(OAc)_2 \cdot 2\ H_2O$ (Merck) und 5.26 g Cs(OAc) gelöst in 140 g warmen destillierten Wasser und 10 ml Eisessig in zwei Teile von 90 ml und 80 ml geteilt und 100 g $SiO_2$-Träger (Siliperl AF125, Fa. Engelhard) wurden bei Raumtemperatur mit dem ersten Anteil getränkt. Der Präkatalysator wurde anschließend 16 h bei 120°C getrocknet, mit dem zweiten Anteil bei Raumtemperatur getränkt, anschließend 16 h bei 120°C getrocknet und schließlich bei 250°C 4 h unter Luft kalziniert.

Der Präkatalysator besaß eine BET-Oberfläche von 175 $m^2/g$ und eine Härte von 47 N/Formkörper. In die oben beschriebene Apparatur wurden ca. 90 ml des Präkatalysators eingefüllt und das Propin/Allen-Gemisch (ca. 63 Vol%ig, 3.3 mmol/min) und Methanol (3.9 mmol/min; Gesamtzulauf mit Inerten: 9.4 mmol/ min; Verhältnis MeOH/ (Propin+Allen)= 1.18) mittels HPLC-Pumpen zudosiert. Die Reaktionstemperatur betrug 200°C und der Druck 1.2 bar (abs), bzw. der Partialdruck der Edukte 0,92 bar. Nach der vollständigen Bildung des Katalysators (ca. 8 h) wurden folgenden Selektivitäten beobachtet: 2-Methoxypropen: 82 %; 2,2-Dimethoxypropan: 5 %; Aceton: 6 %; cis und trans 1-Methoxypropen: 5 %, 1,1-Dimethoxypropan: 1 %. Der Propin/Allen-Umsatz betrug 55 %

Für den fertig gebildeten Katalysator wurden nach dem Ausbau die folgenden Werte ermittelt: BET 222 $m^2/g$; Härte 47 N/Formkörper.

b) (Präkatalysators: Gehalt berechnet als Oxide: 20 % ZnO, 4 % $Li_2O$, 76 % $SiO_2$)

Der Zn/Li/$SiO_2$-Trägerkatalysator wurde durch Imprägnierung von röntgenamorphen $SiO_2$-Formkörpern (Kugeln mit Ø 1.5 - 3.5 mm) mit einer BET-Oberfläche von 300 $m^2/g$, einer Wasseraufnahmekapazität von 0.9 ml/g, und einer Härte von 35 N/Formkörper mit Zinkacetat- und Lithiumacetatlösung hergestellt. Dazu wurde eine Tränklösung bestehend aus 70.97 g $Zn(OAc)_2 \cdot 2\ H_2O$ (Merck) und 8.13 g Li(OAc) gelöst in 140 g warmen destillierten Wasser und 10 ml Eisessig in zwei Teile von 90 ml und 80 ml geteilt und 100 g $SiO_2$-Träger (Siliperl AF125, Fa. Engelhard) wurden bei Raumtemperatur mit dem ersten Anteil getränkt, der Präkatalysator anschließend 16 h bei 120°C getrocknet, mit dem zweiten Anteil bei Raumtemperatur getränkt, anschließend 16 h bei 120°C getrocknet und dann bei 250°C 4 h unter Luft kalziniert.

Der Präkatalysator besaß eine BET-Oberfläche von 210 $m^2/g$ und eine Härte von 46 N/Formkörper. In die oben beschriebene Apparatur wurden ca. 90 ml des Präkatalysators eingefüllt und der Propin/Allen-Gemisch (ca. 63 Vol%ig, 3.3 mmol/min) und Methanol (3.9 mmol/min; Gesamtzulauf mit Inerten: 9.4 mmol/ min; Verhältnis MeOH/(Propin+Allen)= 1.18) wurde mittels HPLC-Pumpen zudosiert. Die Reaktionstemperatur betrug 200°C und der Druck 1.2 bar (abs), bzw. der Partialdruck der Edukte 0,92 bar. Nach der vollständige Bildung des Katalysators (ca. 8 h) wurden folgenden Selektivitäten beobachtet: 2-Methoxypropen: 80 %; 2,2-Dimethoxypropan: 5 %; Aceton: 7 %; cis und trans 1-Methoxypropen: 5 %, 1,1-Dimethoxypropan: 1 %. Der Propin/Allen-Umsatz betrug 54 %.

Für den fertig gebildeten Katalysator wurden nach dem Ausbau die folgenden Werte ermittelt: BET 216 $m^2/g$; Härte 48 N/Formkörper.

c) (Präkatalysator: Gehalt berechnet als Oxide: 17 % ZnO, 3 % $K_2O$, 79,7 % $SiO_2$ und ca. 0,3 % Pd)

Der Zn/K/$SiO_2$-Trägerkatalysator wurde durch Imprägnierung von röntgenamorphen $SiO_2$-Formkörpern (Kugeln mit ∅ 1.5 - 3.5 mm) mit einer BET-Oberfläche von 300 $m^2/g$, einer Wasseraufnahmekapazität von 0.9 ml/g und einer Härte von 35 N/Formkörper mit Zinkacetat- und Kaliumacetatlösung präpariert.

Eine Tränklösung bestehend aus 70.97 g $Zn(OAc)_2 \cdot 2\ H_2O$ (Merck) und 5,26 g K(OAc) gelöst in 140 g warmen destillierten Wasser und 10 ml Eisessig wurde in zwei Teile von 90 ml und 80 ml geteilt und 100 g $SiO_2$-Träger (Siliperl AF125, Fa. Engelhard) wurden bei Raumtemperatur mit dem ersten Anteil getränkt, der Präkatalysator anschließend 16 h bei 120°C getrocknet, mit dem zweiten Anteil bei Raumtemperatur getränkt, anschließend 16 h bei 120°C getrocknet, darauf mit einem 0,3 %igem Pd-Sol imprägniert und erneut 16 h bei 120°C getrocknet.

Der Präkatalysator besaß eine BET-Oberfläche von 169 $m^2$/g und eine Härte von 36 N/Formkörper. In die oben beschriebene Apparatur wurden ca. 90 ml des Präkatalysators eingefüllt und das Propin/Allen-Gemisch (ca. 63 Vol%ig, 3.3 mmol/min) und Methanol (3.9 mmol/min; Gesamtzulauf mit Inerten: 9.4 mmol/ min; Verhältnis MeOH/(Propin+Allen) = 1.18) wurden dann mittels HPLC-Pumpen zudosiert. Die Reaktionstemperatur betrug 200°C und der Druck 1.2 bar (abs), bzw. der Partialdruck der Edukte 0,92 bar. Nach der vollständigen Bildung des Katalysators (ca. 8 h) wurden folgenden Selektivitäten beobachtet: 2-Methoxypropen: 85 %; 2,2-Dimethoxypropan: 4 %; Aceton: 3 %; cis und trans 1-Methoxypropen: 6 %, 1,1-Dimethoxypropan: 1 %. Der Propin/Allen-Umsatz betrug 74 %.
Für den fertig gebildeten Katalysator wurden nach dem Ausbau die folgenden Werte ermittelt: BET 184 $m^2$/g; Härte 38 N/Formkörper.

e) (Präkatalysator: Gehalt berechnet als Oxide: 7,5 % ZnO, 7,5 % CoO, 85 % $SiO_2$)
Der Zn/Co/$SiO_2$-Trägerkatalysator wurde durch Imprägnierung von röntgenamorphen $SiO_2$-Formkörpern (Kugeln mit ∅ 3 - 5 mm) mit einer BET-Oberfläche von 413 $m^2$/g, Wasseraufnahmekapazität von 0.99 ml/g, Härte von 39 N/Formkörper mit Zinkacetat- und Cobalt(II)acetatlösung präpariert.
Eine Tränklösung bestehend aus 23.80 g $Zn(OAc)_2 \cdot 2\ H_2O$ (Merck) und 29.33 g $Co(OAc)_2 \cdot 4\ H_2O$ (Merck) gelöst in 150 g warmen destillierten Wasser wurde in zwei Teile von 95 ml und 85 ml geteilt und 100 g $SiO_2$-Träger (Siliperl AF125, Fa. Engelhard) wurden bei Raumtemperatur mit dem ersten Anteil getränkt. Der Präkatalysator wurde anschließend 16 h bei 120°C getrocknet, mit dem zweiten Anteil bei Raumtemperatur getränkt und erneut 16 h bei 120°C getrocknet. Der Präkatalysator besaß eine BET-Oberfläche von 289 $m^2$/g und eine Härte von 39 N/Formkörper. In der oben beschriebene Apparatur wurden ca. 90 ml des Präkatalysators eingefüllt und das Propin/Allen-Gemisch (ca. 60 Vol%ig, 2.9 mmol/min) und Methanol (2.0 mmol/min; Gesamtzulauf mit Inerten: 7.0 mmol/min; Verhältnis MeOH/(Propin+Allen) = 0.7) wurden dann mittels HPLC-Pumpen zudosiert. Die Reaktionstemperatur betrug 170°C und der Druck 1.2 bar (abs), bzw. einem Partialdruck der Edukte von 0,84 bar. Nach der vollständigen Bildung des Katalysators (ca. 8 h) wurden folgenden Selektivitäten beobachtet: 2-Methoxypropen: 75 %; 2,2-Dimethoxypropan: 8 %; Aceton: 7 %; cis und trans 1-Methoxypropen: 3 %, 1,1-Dimethoxypropan: 1 %. Der Propin/ Allen-Umsatz betrug 28 %.
Für den fertig gebildeten Katalysator wurden nach dem Ausbau die folgenden Werte ermittelt: BET 267 $m^2$/g; Härte 49 N/Formkörper.

Beispiel 10 ($Al_2O_3$-Träger)

100.0 g eines $Al_2O_3$-Trägers (3 mm Stränge, Härte 29.3 N/Formkörper, Wasseraufnahmekapazität 0.71 ml/g, BET-Oberfläche von 185 $m^2$/g) wurden mit 70.0 ml Tetramethoxysilan (Dynasil® , Firma Hüls) imprägniert. Anschließend wurde durch Zugabe von 15 ml konzentrierter Salpetersäure das Tetramethoxysilan hydrolysiert. Der so erhaltene $SiO_2/Al_2O_3$-Träger wurde bei 150°C unter $N_2$ für 15 h getrocknet. Anschließend wurde der Träger mit einer Lösung aus 47.59 g $Zn(OAc)_2 \cdot 2\ H_2O$ (Merck) in 30 g 25 %igem wäßrigem Ammoniak imprägniert (dies entspricht 15 % ZnO auf $SiO_2/Al_2O_3$). Die erhaltenen Stränge wurden dann 16 h bei 120°C getrocknet und 4 h bei 250°C kalziniert. Dieser Präkatalysator hatte eine BET-Oberfläche von 166 $m^2$/g und eine Härte von 54 N/Formkörper.
In die oben beschriebene Apparatur wurden ca. 90 ml des Präkatalysators eingefüllt. Propin/Allen-Gemisch (ca. 52 Vol%ig, 2.9 mmol/min) und Methanol (2.1 mmol/min; Gesamtzulauf mit Inerten: 7.7 mmol/min; Verhältnis MeOH/(Propin+Allen)= 0.71) wurden dann mittels HPLC-Pumpen zudosiert. Die Reaktionstemperatur betrug 170°C und der Druck 1.2 bar (abs., Partialdruck der Edukte 0.78 bar).
Nach einer Induktionszeit von ca. 300 min, in der keine Umsetzung stattfand, setzte die katalytische Aktivität langsam ein.
Nach der vollständigen Bildung des Katalysators (ca. 20 h) wurden folgenden Selektivitäten beobachtet: 2-Methoxypropen: 69 %; 2,2-Dimethoxypropan: 8 %; Aceton: 12 %; cis- und trans-1-Methoxypropen: 8 %, 1,1-Dimethoxypropan: 1 %. Der Propin/Allen-Umsatz betrug 28 %.

Beispiel 11

(Zinkformiat auf Solvay-Träger)

Der Zn/$SiO_2$-Trägerkatalysator wurde durch Imprägnierung von röntgenamorphen $SiO_2$-Formkörpern (Kugeln mit ∅ 3 - 6 mm) mit einer BET-Oberfläche von 358 $m^2$/g, einer Wasseraufnahmekapazität von 0.9 ml/g und einer Härte von 43 N/Formkörper mit ammoniakalischer Zinkformiatlösung, erhalten durch Lösen von Zinkformiat-Dihydrat in $NH_4OH$-haltiger Lösung hergestellt. 100 g $SiO_2$-Träger (Siligel, Fa. Solvay) wurden mit 47.74 g $Zn(HCOO)_2 \cdot H_2O$ gelöst in 120 g 25 %iger $NH_4OH$-Lösung bei Raumtemperatur getränkt, der Präkatalysator anschließend 16 h bei 120°C getrocknet und dann bei 250°C für 4 h unter Luft kalziniert. In der oben beschriebene Apparatur wurden ca. 90 ml des

Präkatalysators eingefüllt. Propin/Allen-Gemisch (ca. 46 Vol%ig, 2.29 mmol/min) und Methanol (2.11 mmol/min; Gesamtzulauf mit Inerten: 7.20 mmol/min; Verhältnis MeOH/(Propin+Allen) = 0.92) wurden dann mittels HPLC-Pumpen zudosiert. Die Reaktionstemperatur betrug 170°C und der Druck 1.20 bar (abs., Partialdruck der Edukte 0.73 bar). Nach der vollständige Bildung des Katalysators (nach ca. 14 h Laufzeit) betrug der Propin/Allen-Umsatz 29 %. Als Hauptprodukte bildeten sich (Selektivitäten bzgl. Propin + Allen in Klammern) 2-Methoxypropen (77 %),2,2-Dimethoxypropan (15 %), 1-Methoxypropen (4 %) und Aceton (3 %).

Beispiel 12

(Zinkacetylacetonat auf Solvay-Träger)

Der Zn/$SiO_2$-Trägerkatalysator wurde durch Imprägnierung von röntgenamorphen $SiO_2$-Formkörpern (Kugeln mit ∅ 3-6 mm) mit einer BET-Oberfläche von 358 $m^2$/g, Wasseraufnahmekapazität von 0.9 ml/g, Härte von 43 N/Formkörper mit methanolischer Zinkacetylacetonat-Lösung, erhalten durch Lösen von Zinkacetylacetonat-Hydrat in Methanol hergestellt.
100 g $SiO_2$-Träger (Siligel, Fa. Solvay) wurden mit jeweils 4 x 90 ml einer Lösung von 80.97 g $Zn(acac)_2 \cdot xH_2O$ (Aldrich) in 360 ml Methanol bei Raumtemperatur getränkt, wobei nach jedem Tränkschritt 16 h bei 120°C getrocknet wurde. Schließlich wurde bei 250°C h unter Luft: kalziniert.
In der oben beschriebene Apparatur wurden ca. 90 ml des Präkatalysators eingefüllt. Propin/Allen-Gemisch (ca. 46 Vol%ig, 2.45 mmol/min) und Methanol (1.83 mmol/min; Gesamtzulauf mit inerten: 7.28 mmol/min; Verhältnis MeOH (Propin+Allen) 0.75) wurden dann mittels HPLC-Pumpen zudosiert. Die Reaktionstemperatur betrug 170°C und der Druck 1.20 bar (abs., Partialdruck der Edukte 0.71 bar). Nach der vollständigen Bildung des Katalysators (nach ca. 14 Stunden) betrug der Propin/Allen-Umsatz 65 %. Als Hauptprodukte bildeten sich (Selektivitäten bzgl. Propin + Allen in Klammern) 2-Methoxypropen (84 %), 2,2-Dimethoxypropan (6 %), 1-Methoxypropen (4 %) und Aceton (2 %).

## Patentansprüche

1. Verfahren zur Herstellung von Verbindungen der Formeln I bzw. II

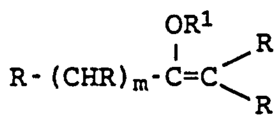 I 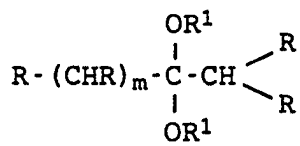 II

in denen $R^1$ Wasserstoff oder einen aliphatischen, cycloaliphatischen, araliphatischen, aromatischen oder heterocyclischen Rest oder einen Acylrest bedeutet, wobei diese Reste weitere Substituenten, die nicht mit Acetylenen oder Allenen reagieren, tragen können und die Reste R unabhängig voneinander für Wasserstoff, oder aliphatische, cycloaliphatische, araliphatische, aromatische oder heterocyclische Reste stehen, die unter Bildung eines Ringes miteinander verbunden sein können und m für 0 oder 1 steht, durch Addition von Verbindungen der Formel III

$$R^1OH \qquad III$$

an Acetylene oder Allene der Formeln IV bzw. V

$$R\text{-}C{\equiv}C\text{-}R \qquad IV$$

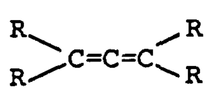 V

wobei $R^1$ und R die oben angegebenen Bedeutungen haben, in der Gasphase bei erhöhter Temperatur in Gegenwart eines heterogenen, ein Silikat enthaltenden Katalysators, **dadurch gekennzeichnet, daß** man einen Katalysator verwendet, der als aktiven Bestandteil ein röntgenamorphes Zink- oder Cadmium-Silikat mit einem Gehalt an Zink oder Cadmium, berechnet als Oxid, von 1 bis 40 Gew.-% enthält, erhältlich durch Aufbringen eines Zink- oder Cadmiumsalzes einer organischen aciden Verbindung, die bei Temperaturen unterhalb von 400°C zersetzbar ist, auf amorphe Kieselsäure und Formierung des Katalysators bei Temperaturen von 50 bis 500°C in Gegenwart einer Hydroxylgruppen enthaltenden Verbindung ausgewählt aus der Gruppe bestehend aus Wasser, Alkanolen mit bis 10 C-Atomen, Diolen oder Polyolen mit 2 bis 6 C-Atomen und 2 bis 3 OH-Gruppen oder Carbonsäuren mit 1 bis 6 C-Atomen.

**2.** Verfahren gemäß Anspruch 1, **dadurch gekennzeichnet, daß** man Zinkacetat, Zinkformiat oder Zinkacetylacetonat verwendet.

**3.** Verfahren gemäß Anspruch 1, **dadurch gekennzeichnet, daß** man einen Katalysator verwendet, dessen katalytisch wirksame Komponente mit bis zu 80 Molprozent, bezogen auf Zink oder Cadmium, noch mit weiteren Metallen dotiert ist ausgewählt aus der Gruppe (A) bestehend aus Natrium, Kalium, Lithium, Rubidium, Cäsium, Beryllium, Magnesium, Calzium, Strontium, Barium, Mangan, Eisen, Kobalt, Nickel und Kupfer, und der Gruppe (B), bestehend aus Titan, Zirkon, Hafnium, Gemanium, Zinn und Blei.

**4.** Verfahren gemäß Anspruch 1, **dadurch gekennzeichnet, daß** man einen Katalysator verwendet, der in Gegenwart des gleichen Alkanols formiert wurde, das mit dem Acetylen oder Allen umgesetzt werden soll.

**5.** Verfahren gemäß Anspruch 1, **dadurch gekennzeichnet, daß** man einen Katalysator verwendet, der durch Tränken von amorpher Kieselsäure mit einem Zinksalz in wässriger oder wässrig-ammoniakalischer Lösung und anschließender Formierung erhältlich ist.

**6.** Verfahren gemäß Anspruch 1, **dadurch gekennzeichnet, daß** man die Addition der Hydroxylgruppen enthaltenden Verbindung $R^1OH$ an die Verbindungen der Formeln IV und V bei Temperaturen von 100 bis 350°C durchführt.

**7.** Verfahren gemäß Anspruch 1, **dadurch gekennzeichnet, daß** man 2-Methoxypropen und/oder 2,2-Dimethoxypropan durch Addition von Methanol an Methylacetylen und/oder Allen bei Temperaturen von 100 bis 350°C herstellt.

**8.** Verfahren zur Herstellung eines für das Verfahren gemäß Anspruch 1 geeigneten röntgenamorphen Zink- oder Cadmiumsilikats, **dadurch gekennzeichnet, daß** man Salze des Zink- oder Cadmiums einer organischen aciden Verbindung, die bei Temperaturen unterhalb von 400°C zersetzbar sind, auf amorphe Kieselsäure durch trockenes Vermischen oder Tränken mit einer Lösung der Salze aufbringt und den Katalysator bei Temperaturen von 50 bis 500°C formiert.

**9.** Verfahren gemäß Anspruch 8, **dadurch gekennzeichnet, daß** man vor der Formierung des Katalysators bei 50 bis 500°C den durch Aufbringen des Zink- oder Cadmiumsalzes auf die amorphe Kieselsäure erhaltenen Präkatalysator einer Calzinierung bei 100 bis 400°C unterwirft, wobei die Temperatur und Verweilzeit bei der Calzinierung so gewählt werden, daß der Präkatalysator noch mindestens 10 Mol% des ursprünglichen Anions der Salze enthält.

**10.** Verfahren gemäß Anspruch 8, **dadurch gekennzeichnet, daß** man ein amorphes Zinksilikat durch Tränken von amorpher Kieselsäure mit einer Zinkacetatlösung und anschließende Formierung gewinnt.

**11.** Röntgenamorpher Zink- oder Cadmiumsilikat-Katalysator, wie er gemäß dem Verfahren des Anspruchs 8 erhalten wird.

## Claims

**1.** A process for the preparation of compounds of the formulae I and II

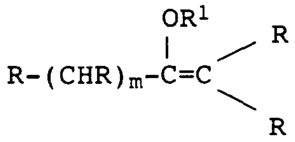 I 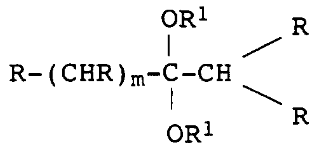 II

where $R^1$ is hydrogen or an aliphatic, cylcoaliphatic, araliphatic, aromatic or heterocyclic radical or an acyl radical, it being possible for these radicals to carry further substituents which do not react with acetylenes or allenes, and radicals R, independently of one another, are hydrogen or aliphatic, cycloaliphatic, araliphatic, aromatic or heterocyclic radicals which may be bonded to one another with formation of a ring, and m is 0 or 1, by addition reaction of a compound of the formula III

$$R^1OH \qquad III$$

with an acetylene or allene of the formula IV or V

$$R\text{-}C\equiv C\text{-}R \qquad IV$$

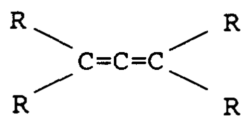 V

where $R^1$ and R have the abovementioned meanings, in the gas phase at elevated temperatures in the presence of a heterogeneous, silicate-containing catalyst, wherein the catalyst used contains, as an active component, an X-ray amorphous zinc silicate or cadmium silicate containing from 1 to 40 % by weight, calculated as oxide, of zinc or cadmium and is obtainable by applying a zinc salt or cadmium salt of an organic acidic compound which is decomposable at below 400°C, to amorphous silica and forming the catalyst at from 50 to 500°C, in the presence of a hydroxyl-containing compound selected from the group consisting of water, alkanols of up to 10 carbon atoms, diols and polyols having 2 to 6 carbon atoms and 2 or 3 OH groups and carboxylic acids of 1 to 6 carbon atoms.

**2.** A process as claimed in claim 1, wherein the zinc acetate, zinc formate or zinc acetylacetonate is used.

**3.** A process as claimed in claim 1, wherein the catalyst used is one whose catalytically active component is furthermore doped with upto 80 mol per cent, based on zinc or cadmium, of further metals selected from the group (A) consisting of sodium, potassium, lithium, rubidium, cesium, beryllium, magnesium, calcium, strontium, barium, manganese, iron, cobalt, nickel and copper, and from the group (B) consisting of titanium, zirconium, hafnium, germanium, tin and lead.

**4.** A process as claimed in claim 1, wherein the catalyst used is one which was formed in the presence of the same alkanol as that to be reacted with the acetylene or allene.

**5.** A process as claimed in claim 1, wherein the catalyst used is one which is obtainable by impregnation of amorphous silica with a zinc salt in aqueous or aqueous ammoniacal solution and subsequent formation.

**6.** A process as claimed in claim 1, wherein the addition reaction of the hydroxyl-containing compound $R^1OH$ with the compounds of the formulae IV and V is carried out at from 100 to 350°C.

**7.** A process as claimed in claim 1, wherein 2-methoxypropene or 2,2-dimethoxypropane is prepared by addition reaction of methanol with methylacetylene or allene at from 100 to 350°C.

**8.** A process for the preparation of an X-ray amorphous zinc silicate or cadmium silicate suitable for a process as claimed in claim 1, wherein salts of zinc or of cadmium with an organic acidic compound which are decomposable at below 400°C are applied to amorphous silica by dry blending or impregnation with a solution of the salts and the catalyst is formed at from 50 to 500°C.

**9.** A process as claimed in claim 8, wherein the precatalyst obtained by applying the zinc or cadmium salt to the amorphous silica is subjected, prior to formation of the catalyst at from 50 to 500°C, to a calcination at from 100 to 400°C, the temperature and the residence time during the calcination being chosen so that the precatalyst still contains at least 10 mol% of the original anion of the salts.

**10.** A process as claimed in claim 8, wherein an amorphous zinc silicate is obtained by impregnation of amorphous silica with a zinc acetate solution and subsequent formation.

**11.** An X-ray amorphous zinc silicate or cadmium silicate catalyst as obtained by the process of claim 8.

## Revendications

**1.** Procédé de préparation de composés ayant les formules I ou II

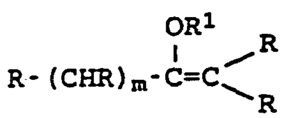 I 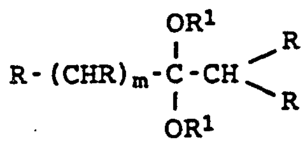 II

dans lesquelles $R^1$ est un atome d'hydrogène ou un radical aliphatique, cycloaliphatique, araliphatique, aromatique ou hétérocyclique, ou encore un radical acyle, ces radicaux pouvant porter d'autres substituants, qui ne réagissent pas avec les acétylènes ou les aliènes, et les radicaux R représentent indépendamment les uns des autres des atomes d'hydrogène ou des radicaux aliphatiques, cycloaliphatiques, araliphatiques, aromatiques ou hétérocycliques, qui peuvent être reliés les uns aux autres avec formation d'un cycle, et m vaut 0 ou 1, par addition de composés de formule III

$$R^1OH \qquad III$$

à des acétylènes ou des allènes, ayant les formules respectivement IV et V

$$R\text{-}C{\equiv}C\text{-}R \qquad IV$$

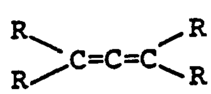 V

dans lesquelles $R^1$ et R ont les significations données ci-dessus, en phase gazeuse à haute température et en présence d'un catalyseur hétérogène contenant un silicate, **caractérisé en ce qu'**on utilise un catalyseur qui en tant que constituant actif contient un silicate de zinc ou de cadmium amorphe aux rayons X, ayant une teneur en zinc ou en cadmium, calculée en oxyde, de 1 à 40 % en poids, et pouvant être obtenue par application, sur une silice amorphe, d'un sel de zinc ou de cadmium d'un composé acide organique pouvant se décomposer à des températures inférieures à 400°C, et façonnage du catalyseur à des températures de 50 à 500°C en présence d'un composé contenant des groupes hydroxyle et choisis dans l'ensemble comprenant l'eau, les alcanols ayant jusqu'à 10 atomes de carbone, les diols ou les polyols ayant de 2 à 6 atomes de carbone et 2 à 3 groupes OH, ou les acides carboxyliques ayant de 1 à 6 atomes de carbone.

**2.** Procédé selon la revendication 1, **caractérisé en ce qu'**on utilise de l'acétate de zinc, du formiate de zinc ou de l'acétylacétonate de zinc.

**3.** Procédé selon la revendication 1, **caractérisé en ce qu'**on utilise un catalyseur dont le composant catalytiquement actif est encore dopé par une quantité allant jusqu'à 80 % en moles, par rapport au zinc ou au cadmium, d'autres métaux choisis dans l'ensemble (A) comprenant le sodium, le potassium, le lithium, le rubidium, le césium, le béryllium, le magnésium, le calcium, le strontium, le baryum, le manganèse, le fer, le cobalt, le nickel et le cuivre, et dans l'ensemble (B) comprenant le titane, le zirconium, le hafnium, le germanium, l'étain et le plomb.

**4.** Procédé selon la revendication 1, **caractérisé en ce qu'**on utilise un catalyseur qui a été façonné en présence du même alcanol que celui qui doit être mis à réagir avec l'acétylène ou l'allène.

**5.** Procédé selon la revendication 1, **caractérisé en ce qu'**on utilise un catalyseur qui peut être obtenu par imprégnation d'une silice amorphe avec un sel de zinc en solution aqueuse ou hydro-ammoniacale, puis façonnage.

**6.** Procédé selon la revendication 1, **caractérisé en ce qu'**on met en oeuvre l'addition du composé $R^1OH$ contenant des groupes hydroxyle sur les composés de formules IV et V à des températures de 100 à 350°C.

**7.** Procédé selon la revendication 1, **caractérisé en ce qu'**on prépare le 2-méthoxypropène et/ou le 2,2-diméthoxypropane par addition de méthanol à du méthylacétylène et/ou à un allène, à des températures de 100 à 350°C.

**8.** Procédé de préparation d'un silicate de zinc ou de cadmium amorphe aux rayons X et convenant au procédé selon la revendication 1, **caractérisé en ce qu'**on applique des sels de zinc ou de cadmium d'un composé acide organique, qui se décomposent à des températures inférieures à 400°C, sur une silice amorphe par mélange ou imprégnation à sec avec une solution des sels, et on façonne le catalyseur à des températures de 50 à 500°C.

**9.** Procédé selon la revendication 8, **caractérisé en ce que**, avant le façonnage du catalyseur, on soumet à une température de 50 à 500°C le précurseur de catalyseur obtenu par application du sel de zinc ou de cadmium sur une silice amorphe à une calcination à une température de 100 à 400°C, la température et le temps de séjour lors de la calcination étant choisis de façon que le précurseur de catalyseur contienne encore au moins 10 % en moles de l'anion initial des sels.

**10.** Procédé selon la revendication 8, **caractérisé en ce qu'**on obtient un silicate de zinc amorphe par imprégnation d'une silice amorphe avec une solution d'acétate de zinc, suivie d'un façonnage.

**11.** Catalyseur à base de silicate de zinc ou de cadmium amorphe aux rayons X, tel qu'obtenu par le procédé selon la revendication 8.

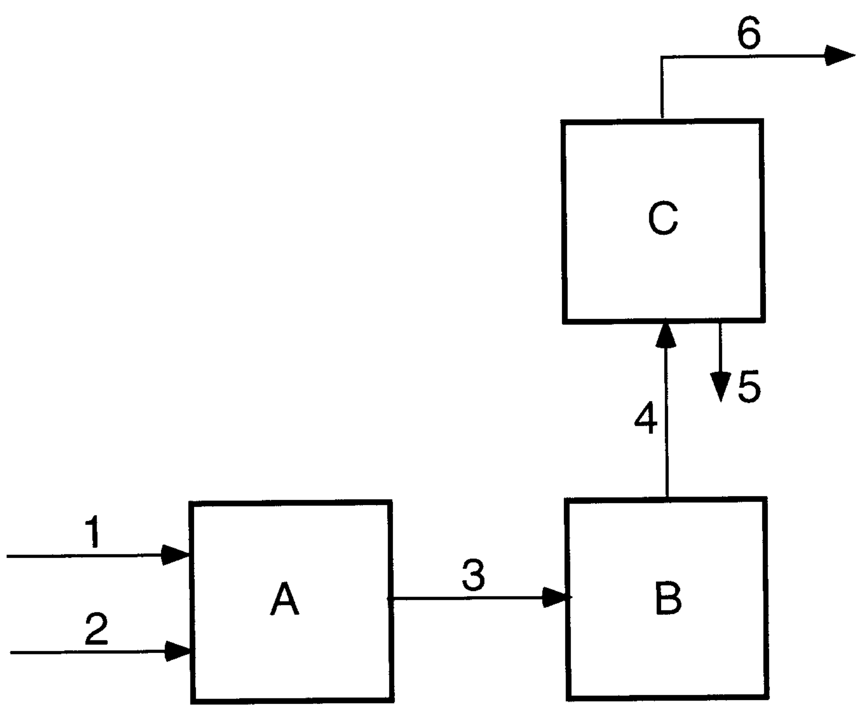
6
C
5
4
1
A
3
B
2